# EUROPEAN PATENT APPLICATION

(11) **EP 2 612 649 A1**
(43) Date of publication of application: **10.07.2013**
(21) Application number: 11821708.2
(22) Date of filing: 29.08.2011
(51) Int. Cl.: A61K 8/22, A61K 8/02, A61Q 5/08, A61Q 5/10

(54) **METHOD FOR DYEING OR BLEACHING HAIR AND KIT FOR DYEING OR BLEACHING HAIR**

(30) Priority: 30.08.2010 JP 2010192729
(71) Applicant: Kao Corporation, Chuo-Ku Tokyo 103-8210 (JP)
(72) Inventor: KODAMA, Daisuke, Tokyo 131-0044 (JP)
(74) Representative: HOFFMANN EITLE
(86) International application number: PCT/JP2011/069396
(87) International publication number: WO 2012/029692

(57) **Abstract**

Provided is a method for hair dyeing or bleaching using a two-part hair dye or bleach kit including a two-part hair dye or bleach composition including a first agent (1) which contains an alkaline agent and a second agent (2) which contains hydrogen peroxide, a container body (12a), and a pump foamer (20) attached to the container body (12a). At least one of the first agent and the second agent contains a foaming agent. A viscosity of liquid mixture (3) is in a range of from 1 to 300 mPa·s at 25°C. A discharge mass of the pump foamer (20) per single depression operation is 1 g or more.

## Description

### Technical Field

The present invention relates to a method for hair dyeing or bleaching. Further, the present invention relates to a hair dye or bleach kit to be used for the method for hair dyeing or bleaching.

### Background Art

A liquid-like or cream-like agent has been in wide use for a long time as a two-part hair dye agent or a two-part hair bleach agent. However, it is difficult operation to evenly apply the agent to hair for a person who is not familiar therewith. Here, a viscosity of the agent to be applied to hair is adjusted to be high on the order of 1000 to 10000 mPa·s to prevent dropping when being left. Accordingly, it is difficult to evenly spread the agent and to sufficiently apply the agent to hair roots. This is the reason of the above. Further, skills such as blocking and using coupled mirrors are required for applying the agent to roots of hair or the back of the head, so that operation thereof takes a lot of time.

Meanwhile, there has been known a product to discharge liquid mixture of a two-part hair dye agent or a two-part bleach agent from a non-aerosol foamer container in a foam-like fashion (e.g., Patent Literature 1). According to this product, the liquid mixture can be applied easily and evenly to hair even by a person who is not familiar therewith as discharging the liquid mixture of a first agent and a second agent from the non-aerosol foamer container in a foam-like fashion. As a result, color unevenness is less likely to occur on finish. Further, according to the product, since the liquid mixture can be easily applied, skills such as blocking and using coupled mirrors are not required and the time required for the operation is much shortened compared to a traditional product. Owing to the above advantages, the products disclosed in the literature have been commercially successful as obtaining a large market share in spite of being launched onto the market relatively recently.

There have been known technologies to smoothly perform operation of a two-part hair dye agent or a two-part hair bleach agent while a squeeze foamer is selected as the non-aerosol foamer container used for the product disclosed in Patent Literature 1 (see Patent Literatures 2 and 3). In the above technologies, foam quantity of liquid mixture to be discharged with single squeezing is about 3 g or more being appropriate quantity for being placed on one hand. This quantity is relatively large for squeeze quantity of a squeeze foamer. Regardless of the above, according to the technologies disclosed in the literatures, it is possible to obtain advantages that foam quality of liquid mixture after repeating squeezing can be extremely fine from start to end of squeezing and that the number of squeezing repetition can be decreased for obtaining a total discharge mass required for applying to entire hair.

Meanwhile, a pump foamer is widely known as a non-aerosol foamer container and is widely used as a container for daily-use products such as foamy-like cleaner for hand-wash and cleansing foam. Compared to a squeeze foamer, a pump foamer has an advantage that a foam discharge mass per single discharge operation is more likely to be constant. Further, there is also an advantage that foam with a gas-liquid mixing ratio being in a constant range is more likely to be obtained from start to end of discharge operation. However, most of pump foamers have a small foam discharge mass per single depression operation as being about 0.5 g. A pump foamer with a foam discharge mass per single depression operation being 1 g or more is known but is not used so much. This is considered to be because of reasons (a) and (b) described below.

(a) Foam discharge volume per single discharge operation is determined from foam discharge mass (g) per single discharge operation and a gas-liquid mixing ratio (mL/g). Here, the gas-liquid mixing ratio cannot be largely varied from a viewpoint of usability of the obtained foam. Therefore, the foam discharge volume is to be adjusted mainly by the discharge mass.

(b) Meanwhile, the foam discharge volume per single discharge operation is proportional to volume of an air chamber of the pump foamer. Therefore, to increase the discharge volume per one time, it is required to increase the volume of the air chamber of the pump foamer. To increase the volume of the air chamber, it is required to satisfy either or both of (1) increasing height of the air chamber and (2) enlarging a diameter of a traverse section of the air chamber. However, in a case of (1), since depressing distance (stroke) of one time is lengthened by the amount thereof, the height cannot be increased so much from a viewpoint of usability. In a case of (2), since an area of a traverse section of the entire container becomes large, the diameter cannot be enlarged so much from viewpoints of transportability, usability and the like of products with the container.

In view of the above, a pump foamer which is used widely and generally has foam discharge mass per single depression operation being about 0.5 g. Therefore, in a case of using generous quantity of foam at one time, the number of pushing a pump head becomes large. In a case of foamy-like cleaner for hand-wash or cleansing foam, since foam mass per one time of washing or cleansing is about 1 g at most, the number of pushing the pump head may be small as being about 2 to 3 times. Since a user is not burdened so much with such a level of times, the above has not become obvious until now as a problem to be solved.

Meanwhile, for applying a two-part hair dye agent to hair using a pump foamer, considerable quantity of foam is to be discharged in a short time. Here, the present inventor recognized the above as a problem to increase physical burden for a user.

Further, since a general pump foamer is designed with an eye on pushing a pump head several times in a short time, depression pressure of the pump head is set to be considerably high by strengthening a spring so that the pump head is instantly returned to an original position before depression when depressing force of the pump head is released after the pump head is completely depressed. In a case of a product with which large quantity of an agent on the order of 100 g for one time of entire dyeing operation is required to be applied to hair in a short time being about 5 to 10 minutes like a two-part hair dye agent, it is required to push the pump head 100 times or more in a short time. In such a case, a physical burden for a user becomes larger.

In addition, for reducing a physical burden applied to a user with solving means described below, the present inventor further recognized another problem.

### Citation List

### Patent Literature

Patent Literature 1: US 2004/0213752 A1
Patent Literature 2: WO 2008/136441 A1
Patent Literature 3: WO 2008/136433 A1

### Summary of Invention

### Technical Problem

As described above, a technology to smoothly use a dye agent is proposed for a two-part foam-like hair dye agent using a squeeze foamer. However, it is a reality that a technology to smoothly use a dye agent is not proposed for a two-part foam-like hair dye agent using a pump foamer. Accordingly, an object of the present invention is to provide a technology to smoothly use a two-part foam-like hair dye agent using a pump foamer.

### Solution to Problem

To solve the above-mentioned problem, the present inventor recognized that selecting a pump foamer which discharges foam with mass being in a specific range per single depression operation provides smooth usability of the pump foamer as a foam discharge container for a two-part foam-like hair dye agent through a hard study thereof, so that the present invention was completed.
Further, the present inventor recognized that using the pump foamer which discharges foam with mass being in a specific range per single depression operation causes a new problem which is previously non-existent as a foam discharge container for a two-part foam-like hair dye agent. Then, the present inventor recognized that adopting a pump foamer having a specific configuration as means to solve the new problem provides smooth usability of the pump foamer as a foam discharge container for a two-part foam-like dye agent, so that the present invention was completed.

The present invention relates to a method for hair dyeing or bleaching using a two-part hair dye or bleach kit. The two-part hair dye or bleach kit includes a two-part hair dye or bleach composition including a first agent which contains an alkaline agent and a second agent which contains hydrogen peroxide, a container body in which liquid mixture of the first agent and the second agent is to be stored, and a pump foamer which is to be attached to the container body and which discharges the liquid mixture in a foam. At least one of the first agent and the second agent contains a foaming agent, a viscosity of the liquid mixture is in a range of 1 to 300 mPa·s at 25°C, and a discharge mass of the pump foamer per single depression operation is 1 g or more. The method includes steps of A) mixing the first agent and the second agent in the container body, B) discharging foam of the liquid mixture by depressing a pump head of a pump foamer container in which the pump foamer is attached to the container body, C) applying the discharged foam of the liquid mixture to hair, D) allowing the liquid mixture applied to hair to stand for 3 to 60 minutes, and E) washing away the liquid mixture applied to hair.

The present invention includes following subjects.
[1] A method for hair dyeing or bleaching using a two-part hair dye or bleach kit comprising:
   a two-part hair dye or bleach composition including a first agent which contains an alkaline agent and a second agent which contains hydrogen peroxide,
   a container body in which liquid mixture of the first agent and the second agent is to be stored, and
   a pump foamer which is to be attached to the container body and which discharges the liquid mixture in a foam,
   wherein at least one of the first agent and the second agent contains a foaming agent,
   a viscosity of the liquid mixture is in a range of from 1 to 300 mPa·s at 25°C,
   a discharge mass of the pump former per single depression operation is 1 g or more, and
   the method includes steps of:
      A) mixing the first agent and the second agent in the container body;
      B) discharging foam of the liquid mixture by depressing a pump head of a pump foamer container in which the pump foamer is attached to the container body;
      C) applying the discharged foam of the liquid mixture to hair;
      D) allowing the liquid mixture applied to hair to stand for 3 to 60 minutes; and
      E) washing away the liquid mixture applied to hair.

[2] The method for hair dyeing or bleaching according to subject [1], wherein the kit includes a container body in which the first agent is filled, a container body in which the second agent is filled, the container body in which the liquid mixture of the first agent and the second agent is to be stored, and the pump foamer which is to be attached to the container body storing the liquid mixture, and
   the pump foamer container is completed by attaching the pump foamer to the container body storing the liquid mixture.

[3] The method for hair dyeing or bleaching according to subject [1], wherein the kit includes a container body in which the first agent is filled, a container body in which the second agent is filled, and the pump foamer which is to be attached to the container body in which the second agent is filled, and
   the pump foamer container is completed by pouring the first agent into the container body in which the second agent is filled, followed by attaching the pump foamer to the container body.

[4] The method for hair dyeing or bleaching according to any one of subjects [1] to [3], wherein foam of the liquid mixture is discharged by depressing a pump head with one hand and the discharged foam is received by the other hand in the step (B).

[5] The method for hair dyeing or bleaching according to any one of subjects [1] to [4], wherein foam is applied to hair with a hand in the step (C).

[6] The method for hair dyeing or bleaching according to any one of subjects [1] to [5], wherein operation to discharge foam with 1 to 5 times of depression of the pump head after the foam discharged with 1 to 5 times of depression of the pump head is applied to hair is repeated in the step (B).

[7] The method for hair dyeing or bleaching according to any one of subjects [1] to [6], wherein a discharge port of the pump foamer container is formed to have an angle θ between a perpendicular line of an opening face of the discharge port and the vertical direction being in a range of from 0° to 70° in a state that the pump foamer container is upright.

[8] The method for hair dyeing or bleaching according to subject [7], wherein the angle θ is in a range of from 10° to 60°.

[9] The method for hair dyeing or bleaching according to subject [7], wherein the angle θ is in a range of from 20° to 50°.

[10] The method for hair dyeing or bleaching according to any one of subjects [1] to [9], wherein an area of a discharge port of the pump foamer container is in a range of from 45 to 200 mm².

[11] The method for hair dyeing or bleaching according to any one of subjects [1] to [10], wherein a maximum load until a pump head of the pump foamer container is completely depressed in a state that the liquid mixture of the first agent and the second agent is stored in the container body is in a range of from 10 to 25 N.

[12] The method for hair dyeing or bleaching according to subject [11], wherein the maximum load is in a range of from 12 to 23 N.

[13] The method for hair dyeing or bleaching according to subject [11], wherein the maximum load is in a range of from 15 to 22 N.

[14] The method for hair dyeing or bleaching according to any one of subjects [1] to [13], wherein a pump head of the pump foamer container takes time for from 0.2 to 3 seconds until being returned to an original position before depression when a depressed state of the pump head is released in a state that the liquid mixture of the first agent and the second agent is stored in the container body after the pump head is completely depressed.

[15] The method for hair dyeing or bleaching according to subject [14], wherein the time until the pump head is returned to the original position before depression is in a range of from 0.4 to 2 seconds.

[16] The method for hair dyeing or bleaching according to subject [14], wherein the time until the pump head is returned to the original position before depression is in a range of from 0.6 to 1.5 seconds.

[17] The method for hair dyeing or bleaching according to any one of subjects [1] to [16], wherein an air chamber of the pump foamer of the pump foamer container exists outside the container body.

[18] The method for hair dyeing or bleaching according to any one of subjects [1] to [17], wherein foam of the liquid mixture is applied to unblocked hair in the step (C).

[19] The method for hair dyeing or bleaching according to subject [18], wherein the foam is further rubbed with fingers after being applied in the step (C).

[20] The method for hair dyeing or bleaching according to any one of subjects [1] to [19], wherein the pump foamer discharges a mass of 8 g or less per single depression operation.

[21] The method for hair dyeing or bleaching according to subject [20], wherein the pump foamer discharges a mass of 6 g or less per single depression operation.

[22] The method for hair dyeing or bleaching according to subject [20], wherein the pump foamer discharges a mass of 4 g or less per single depression operation.

[23] The method for hair dyeing or bleaching according to any one of subjects [1] to [22], wherein the pump foamer discharges a mass of 1.5 g or more per single depression operation.

[24] The method for hair dyeing or bleaching according to subject [23], wherein the pump foamer discharges a mass of 2 g or more per single depression operation.

[25] The method for hair dyeing or bleaching according to subject [23], wherein the pump foamer discharges a mass of 2.5 g or more per single depression operation.

[26] The method for hair dyeing or bleaching according to any one of subjects [1] to [25], wherein the foaming agent is a surface-active agent which is selected from the group consisting of a nonionic surface-active agent, an anionic surface-active agent, a cationic surface-active agent, an amphoteric surface-active agent, and a semipolar surface-active agent.

[27] The method for hair dyeing or bleaching according to subject [26], wherein contained quantity of the surface-active agent in the liquid mixture of the first agent and the second agent is in a range of 0.1 % to 15% by mass.

[28] The method for hair dyeing or bleaching according to any one of subjects [1] to [27], wherein at least one of the first agent and the second agent contains higher alcohol having 14 to 24 carbon atoms.

[29] The method for hair dyeing or bleaching according to any one of subjects [1] to [28], wherein pH of the liquid mixture of the first agent and the second agent is in a range of from 8 to 12.

[30] The method for hair dyeing or bleaching according to any one of subjects [1] to [29], wherein the liquid mixture is discharged from the pump foamer container with a gas-liquid mixing ratio being in a range of from 7 to 40 mL/g in a foam-like fashion in the step (B).

[31] The method for hair dyeing or bleaching according to subject [30], wherein the gas-liquid mixing ratio is in a range of from 10 to 30 mL/g.

[32] The method for hair dyeing or bleaching according to any one of subjects [1] to [31], wherein two foam homogenizing members formed of mesh sheets are arranged at a discharge passage for the liquid mixture at the pump head of the pump foamer container, and the mesh sheet at a closer side to a discharge port of the pump foamer container has apertures in a range of from 150 to 280 mesh and the mesh sheet at a farther side from the discharge port (a closer side to the air chamber) has apertures in a range of from 50 to 220 mesh.

[33] The method for hair dyeing or bleaching according to any one of subjects [1] to [32], wherein total quantity of the first agent and the second agent is in a range of from 50 to 1000 mL.

[34] The method for hair dyeing or bleaching according to any one of subjects [1] to [33], wherein an area of a bottom face of the container body of the pump foamer container is in a range of from 20 to 60 cm².

[35] The method for hair dyeing or bleaching according to any one of subjects [1] to [34], wherein the bottom face of the container body of the pump foamer container has approximately square shape.

[36] The method for hair dyeing or bleaching according to any one of subjects [1] to [35], wherein the pump foamer container is a floor-standing type.

[37] A two-part hair dye or bleach kit comprising:
   a two-part hair dye or bleach composition including a first agent which contains an alkaline agent and a second agent which contains hydrogen peroxide;
   a container body in which liquid mixture of the first agent and the second agent is to be stored; and
   a pump foamer which is to be attached to the container body and which discharges the liquid mixture in a foam,
   wherein a pump foamer container is completed by attaching the pump foamer to the container body,
   at least one of the first agent and the second agent contains a foaming agent,
   a viscosity of the liquid mixture is in a range of from 1 to 300 mPa·s at 25°C, and
   a discharge mass of the pump foamer container per single depression operation is 1 g or more.

[38] The two-part hair dye or bleach kit according to subject [37], further comprising:
   a container body in which the first agent is filled;
   a container body in which the second agent is filled;
   the container body in which the liquid mixture of the first agent and the second agent is to be stored; and
   the pump foamer which is to be attached to the container body,
   wherein the pump foamer container is completed by attaching the pump foamer to the container body storing the liquid mixture of the first agent and the second agent.

[39] The two-part hair dye or bleach kit according to subject [37], further comprising:
   a container body in which the first agent is filled;
   a container body in which the second agent is filled; and
   the pump foamer which is to be attached to the container body in which the second agent is filled,
   wherein the pump foamer container is completed by pouring the first agent into the container body in which the second agent is filled, followed by attaching the pump foamer to the container body.

[40] The two-part hair dye or bleach kit according to any one of subjects [37] to [39], wherein a discharge port of the pump foamer container is formed to have an angle θ between a perpendicular line of an opening face of the discharge port and the vertical direction being in a range of from 0° to 70° in a state that the pump foamer container is upright.

[41] The two-part hair dye or bleach kit according to subject [40], wherein the angle θ is in a range of from 10° to 60°.

[42] The two-part hair dye or bleach kit according to subject [40], wherein the angle θ is in a range of from 20° to 50°.

[43] The two-part hair dye or bleach kit according to any one of subjects [37] to [42], wherein an area of the discharge port of the pump foamer container is in a range of from 45 to 200 mm².

[44] The two-part hair dye or bleach kit according to any one of subjects [37] to [43], wherein a maximum load until a pump head of the pump foamer container is completely depressed in a state that the liquid mixture of the first agent and the second agent is stored in the container body is in a range of from 10 to 25 N.

[45] The two-part hair dye or bleach kit according to subject [44], wherein the maximum load is in a range of from 12 to 23 N.

[46] The two-part hair dye or bleach kit according to subject [44], wherein the maximum load is in a range of from 15 to 22 N.

[47] The two-part hair dye or bleach kit according to any one of subjects [37] to [46], wherein a pump head of the pump foamer container takes time for from 0.2 to 3 seconds until being returned to an original position before depression when a depressed state of the pump head is released in a state that the liquid mixture of the first agent and the second agent is stored in the container body after the pump head is completely depressed.

[48] The two-part hair dye or bleach kit according to subject [47], wherein the time until the pump head is returned to the original position before depression is in a range of from 0.4 to 2 seconds.

[49] The two-part hair dye or bleach kit according to subject [47], wherein the time until the pump head is returned to the original position before depression is in a range of from 0.6 to 1.5 seconds.

[50] The two-part hair dye or bleach kit according to any one of subjects [37] to [49], wherein an air chamber of the pump foamer of the pump foamer container exists outside the container body.

[51] The two-part hair dye or bleach kit according to any one of subjects [37] to [50], wherein a discharge mass per single depression operation of the pump foamer is 8 g or less.

[52] The two-part hair dye or bleach kit according to subject [51], wherein a discharge mass per single depression operation of the pump foamer is 6 g or less.

[53] The two-part hair dye or bleach kit according to subject [51], wherein a discharge mass per single depression operation of the pump foamer is 4 g or less.

[54] The two-part hair dye or bleach kit according to any one of subjects [37] to [53], wherein a discharge mass per single depression operation of the pump foamer is 1.5 g or more.

[55] The two-part hair dye or bleach kit according to subject [54], wherein a discharge mass per single depression operation of the pump foamer is 2 g or more.

[56] The two-part hair dye or bleach kit according to subject [55], wherein discharge mass per single depression operation of the pump foamer is 2.5 g or more.

[57] The two-part hair dye or bleach kit according to any one of subjects [37] to [56], wherein the foaming agent is a surface-active agent which is selected from a group of a nonionic surface-active agent, an anionic surface-active agent, a cationic surface-active agent, an amphoteric surface-active agent, and a semipolar surface-active agent.

[58] The two-part hair dye or bleach kit according to subject [57], wherein contained quantity of the surface-active agent in the liquid mixture of the first agent and the second agent is in a range of from 0.1% to 15% by mass.

[59] The two-part hair dye or bleach kit according to any one of subjects [37] to [58], wherein at least either of the first agent and the second agent contains higher alcohol having 14 to 24 carbon atoms.

[60] The two-part hair dye or bleach kit according to any one of subjects [37] to [59], wherein pH of the liquid mixture of the first agent and the second agent is in a range of from 8 to 12.

[61] The two-part hair dye or bleach kit according to any one of subjects [37] to [60], wherein a gas-liquid mixing ratio of foam-like liquid mixture discharged from the pump foamer container is in a range of from 7 to 40 mL/g.

[62] The two-part hair dye or bleach kit according to subject [61], wherein the gas-liquid mixing ratio is in a range of from 10 to 30 mL/g.

[63] The two-part hair dye or bleach kit according to any one of subjects [37] to [62], wherein two homogenizing members formed of mesh sheets are arranged at a discharge passage for the liquid mixture at the pump head of the pump foamer container, and the mesh sheet at a closer side to a discharge port of the pump foamer container has apertures in a range of from 150 to 280 mesh and the mesh sheet at a farther side from the discharge port (a closer side to the air chamber) has apertures in a range of from 50 to 220 mesh.

[64] The two-part hair dye or bleach kit according to any one of subjects [37] to [63], wherein total quantity of the first agent and the second agent is in a range of from 50 to 1000 mL.

[65] The two-part hair dye or bleach kit according to any one of subjects [37] to [64], wherein an area of a bottom face of the container body of the pump foamer container is in a range of from 20 to 60 cm².

[66] The two-part hair dye or bleach kit according to any one of subjects [37] to [65], wherein the bottom face of the container body of the pump foamer container has approximately square shape.

[67] The two-part hair dye or bleach kit according to any one of subjects [37] to [66], wherein the pump foamer container is a floor-standing type.

### Advantageous Effects of Invention

The present invention provides a method and a kit for smoothly using a two-part foam-like agent for hair dyeing or hair bleaching using a pump foamer.

### Brief Description of Drawings

[FIG. 1] FIGS. 1(a) to 1(d) are schematic views illustrating an embodiment of a usage method of a two-part kit for hair dyeing or hair bleaching of the present invention.
[FIG. 2] FIGS. 2(a) and 2(b) are schematic views illustrating another embodiment of a usage method of a two-part kit for hair dyeing or hair bleaching of the present invention.
[FIG. 3] FIGS. 3(a) and 3(b) are schematic views illustrating a state of discharging foam of liquid mixture of a first agent and a second agent.
[FIG. 4] FIG. 4 is an enlarged view of a main part of a pump foamer of a pump foamer container used in the present invention.
[FIG. 5] FIG. 5 is a schematic view illustrating a poor state of discharging foam of the liquid mixture of the first agent and the second agent.
[FIG. 6] FIG. 6 is a perspective view illustrating another example of the pump foamer container used in the present invention.

### Description of Embodiments

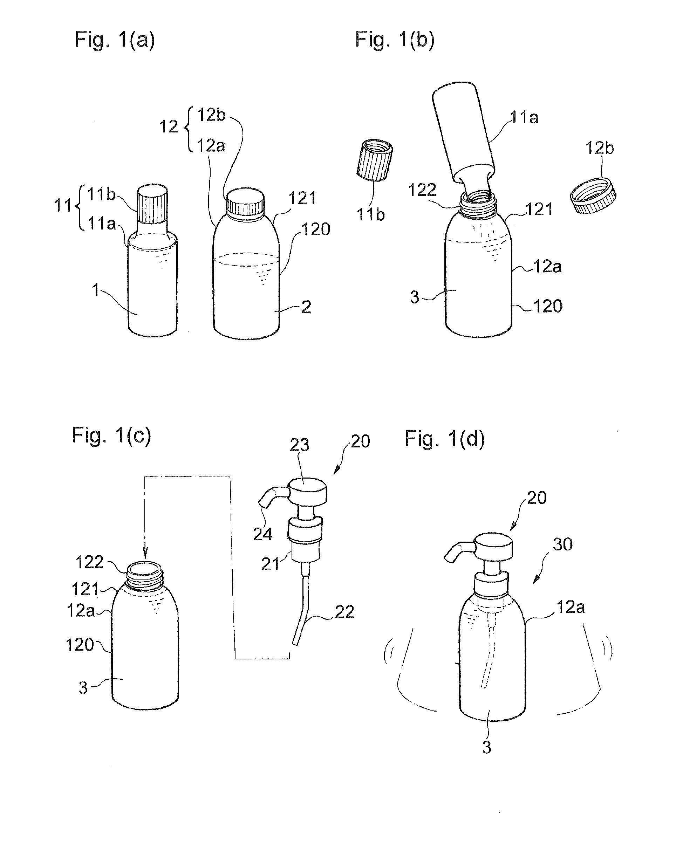

In the following, preferable embodiments of the present invention will be described with reference to the drawings. In this specification, various numerical values denote values measured at room temperature unless otherwise specified. The room temperature denotes 25°C. FIGS. 1(a) to 1(d) illustrate an embodiment of a first usage method of a two-part hair dye or hair bleach kit for (hair beauty preparation) of the present invention. The hair dye or hair bleach kit (hereinafter, collectively called hair beauty preparation kit) of the present embodiment includes a first agent 1 containing an alkaline agent and a second agent 2 containing hydrogen peroxide.

In the present invention, a two-part hair dye or hair bleach composition conceptually includes a composition for dyeing or bleaching formed of the first agent 1 and the second agent 2 to be used after being mixed at the time of usage. As for dyeing, the first agent 1 contains an alkaline agent and a dye and the second agent 2 contains hydrogen peroxide. As for bleaching, the first agent 1 contains an alkaline agent without containing a dye and the second agent 2 contains hydrogen peroxide. In any of cases for dyeing and bleaching, a composition used in the present invention also conceptually includes a case of using a third agent which contains persulfate. In such a case, the first agent, the second agent, and the third agent are used after being mixed.

The first agent 1 is filled in a first container 11. The second agent 2 is filled in a second container 12 which is larger than the first container 11. The first container 11 includes a container body 11 a and a cover body 11b which is screwed with a mouth portion of the container body 11a. Similarly, the second container 12 includes a container body 12a and a cover body 12b which is screwed with a mouth portion of the container body 12a. As described later, the container body 12a of the second container 12 serves as a container body of a pump foamer container as well.

The container body 12a of the second container 12 includes a barrel portion 120, a shoulder portion 121 which is smoothly continued from an upper part of the barrel portion 120, and a mouth-neck portion 122 which is opened upward at an upper part of the shoulder portion 121. The barrel portion 120 is formed to have the same shape in the height direction with a shape of a bottom face thereof. The bottom face shape of the barrel portion 120 may be circular, oval, or approximately rectangular, for example. Generally, from a viewpoint of easiness of printing on the entire container, the bottom face shape is circular or oval in many cases. The shoulder portion 121 has a traverse section which is shaped similarly to the bottom face shape of the barrel portion 120. The traverse section of the shoulder portion 121 is gradually shrunk in diameter toward the upper side.

To prevent oxidation of the dye and volatilization of ammonia contained in the first agent 1, it is preferable that the first container 11 is made of gas-permeation-proof material. Meanwhile, to prevent pressure increase in the second container 12 owing to oxygen caused by resolution of the hydrogen peroxide contained in the second agent, it is preferable that the second container 12 is made of gas-permeable material.

The hair beauty preparation kit of the present embodiment further includes a pump foamer 20. In a state before usage of the hair beauty preparation kit, the pump foamer 20 is in a separate state without being coupled with the container body.

When using the hair beauty preparation kit of the present embodiment, first, the cover body 11b of the first container 11 in a state illustrated in FIG. 1(a) is detached and the cover body 12b of the second container 12 is detached as well. Then, as illustrated in FIG. 1(b), the container body 11a of the first container 11 is inverted and the mouth portion thereof is inserted to the mouth portion of the container body 12a of the second container 12. Subsequently, the first agent 1 filled in the container body 11a of the first container 11 is poured into the container body 12a of the second container 12 and the first agent 1 and the second agent 2 are mixed. In this case, since a traverse section of the barrel portion of the container body 11a of the first container 11 is larger than a traverse section of the mouth portion of the container body 12a of the second container 12 as illustrated in FIG. 1(b), insertion of the container body 11a of the first container 11 is restricted by the mouth portion of the container body 12a of the second container 12. As a result, the container body 11 a of the first container 11 is prevented from unintentionally falling into the container body 12a of the second container 12 when pouring the first agent 1 into the second agent.

As a result of pouring the first agent 1 into the second agent 2, liquid mixture 3 of the first agent 1 and the second agent 2 is stored in the container body 12a of the second container 12. In the present invention, since a viscosity of the first agent 1 and the second agent 2 is much lower than that of a gel-like or cream-like agent type as described later, mixing is performed to some extent only by storing the first agent 1 and the second agent 2 in the same container. Accordingly, as described later, even in a state before performing mixing operation, the first agent 1 and the second agent 2 stored in the same container is called the liquid mixture 3 for convenience sake. Next, as illustrated in FIG. 1(c), the pump foamer 20 is inserted through the mouth portion of the container body 12a of the second container 12 which stores the liquid mixture 3. The pump foamer 20 is used for discharging liquid mixture 3 stored in the container body 12a in a foam-like fashion. The pump foamer 20 includes an air chamber 21, a suction tube 22 which droops from a bottom part of the air chamber 21, a pump head 23 continuously formed from an upper part of the air chamber 21, and the like. A discharge passage (not illustrated) of the liquid mixture 3 is formed in the pump head 23. One end of the discharge passage is connected to the air chamber 21. The other end of the discharge passage is opened at a discharge port 24. Further, a foam generating member and a foam homogenizing member (not illustrated for both) for foaming the liquid mixture 3 is placed in the discharge passage. For example, the foam homogenizing member is structured with a mesh sheet, a porous body, or the like. The foam homogenizing member may be used singularly or plurally as being two or more. For example, in a case of using two mesh sheets as the foam homogenizing member, a mesh sheet at a closer side to the air chamber 21, that is, a farther side from the discharge port 24, may be set to have apertures in a range of 50 to 220 mesh preferably, 90 to 195 mesh more preferably, and 130 to 170 mesh even more preferably. Meanwhile, a mesh sheet at a closer side to the discharge port 24 may be set to have apertures in a range of 150 to 280 mesh preferably, 165 to 250 mesh more preferably, and 180 to 220 mesh even more preferably. Here, mesh denotes the number of apertures per one inch. The pump foamer 20 having the above-mentioned structure adopts a structure which is similar to a traditionally known structure without being specifically restricted.

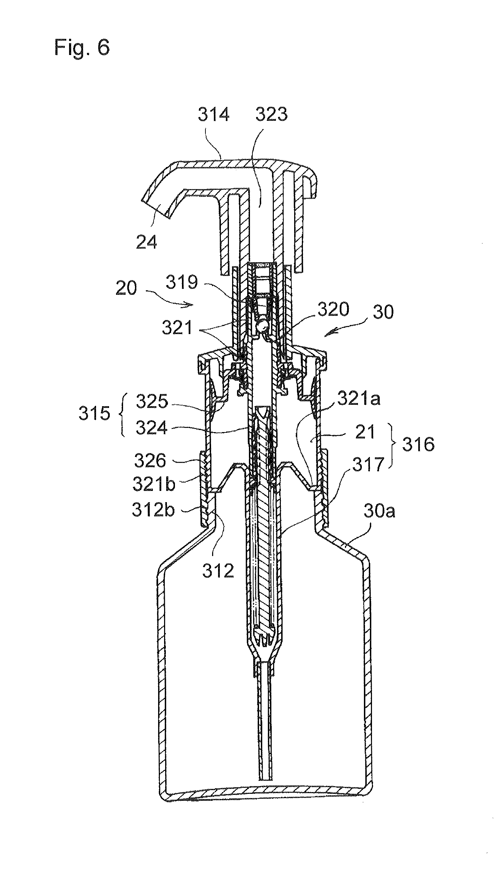

The pump foamer container including the pump foamer 20 is set to have larger discharge mass per single depression operation than discharge mass of a traditional pump foamer container. Specifically, the discharge mass per single depression operation is set to be 1 g or larger, 1.5 g or larger preferably, 2 g or larger more preferably, and 2.5 g or larger even more preferably. Setting the discharge mass of the pump foamer container to be larger provides following advantages. For example, in a case to dye or bleach entire semi-long hair (having length to the extent of reaching a shoulder), it is estimated that quantity of the liquid mixture 3 necessary for the above is about 100 g. Here, dyeing or bleaching operation is required to be completed before chemical reaction between the first agent 1 and the second agent 2 proceeds. Specifically, it is required to be completed in a short time within about 5 to 10 minutes after mixing. Accordingly, to discharge the liquid mixture 3 of about 100 g from the pump foamer container in a short time, it is advantageous to discharge targeted quantity of foam with the less number of depression operation by enlarging discharge mass per one time. From a viewpoint of the above, the discharge mass of the pump foamer container per single depression operation is set to the above-mentioned value or larger in the present invention. The more the discharge mass per single depression operation is increased, the more it is advantageous for discharging the targeted mass of foam with the less number of depression operation. However, on the other hand, it is required to enlarge capacity of the air chamber 21 in the pump foamer 20. Enlarged capacity of the air chamber 21 requires enlarging of capacity of the pump foamer container and further requires enlarging of capacity of the hair beauty preparation kit. Further, when discharge quantity per single depression operation is simply enlarged, it becomes hard to be used owing to increased depression pressure due to increased resistance of liquid mixture when depressing the pump head. For smooth using of the foam-like dye agent of the present invention, it is preferable that foam discharged from the foamer container is applied to hair after once being put on one hand, and accordingly, it is preferable that foam obtained with single depression operation is in a range to be capable of being held by one hand. From the above-mentioned viewpoints, an upper limit value of the discharge mass per single depression operation is set to be 8 g preferably, 6 g more preferably, and 4 g even more preferably. Here, the discharge mass per single depression operation can be adjusted by capacity of a liquid chamber (a portion indicated by a numeral 317 in FIG. 6 described later) of the pump foamer 20.

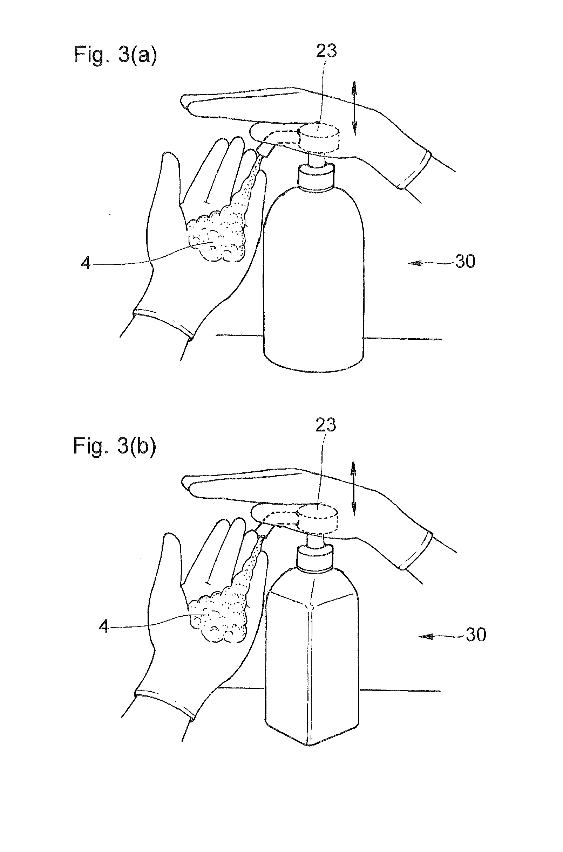

Returning to FIG. 1, lastly, the pump foamer 20 is screwed and attached to the container body 12a of the second container 12 in a hermetically closed fashion to complete the pump foamer container 30, as illustrated in FIG. 1(d). In this state, the air chamber 21 of the pump foamer 20 is located in the container body 12a. The pump foamer container 30 may be a floor-standing type as illustrated in FIG. 3 described below or a handy type to discharge foam in a state of being held by hand. From a viewpoint of capability to perform discharging operation and applying operation of foam being a dye agent repeatedly and smoothly (especially with both hands) as having a convenient size to be used for a two-part dye agent, it is preferable that the pump foamer container 30 is a floor-standing type which does not require to be held by hand. After the pump foamer container 30 is completed, mixing of the first agent 1 and the second agent 2 is accelerated. Not being specifically limited, examples of a mixing method include a method to lightly shake the pump foamer container 30 just like shaking a test tube, for example as illustrated in FIG. 1(d). Here, the mixing can also be performed before attaching the pump foamer 20 to the container body 12a of the second container 12. In this case, it is preferable to perform mixing after hermetical closing with the cover body 12b of the second container 12 and to attach the pump foamer 20 after mixing. The attaching process of the pump foamer 20 and the mixing process may be performed in either sequence also in a case that another mixing method described below is adopted. Here, from a viewpoint of capability of eliminating open-close operation of the cover body 12b, it is preferable to perform mixing after the pump foamer 20 is attached.

As another mixing method, it is possible to adopt a mixing method to sharply shake up and down the pump foamer container 30. Alternatively, it is also possible to adopt a mixing method in which the pump foamer container 30 is put into an inverted state or a falling-over state from an approximately upright state and returned again into the appropriately upright state. Specifically, it is preferable to perform cycles in which the pump foamer container 30 is put into an inverted or falling-over state from an approximately upright state and returned again into the appropriately upright state at a speed in a range of 1 to 30 times per 10 seconds, 1.5 to 20 times per 10 seconds more preferably, and 2 to 10 times per 10 seconds even more preferably. The operation of putting from an approximately upright state to an inverted or falling-over state and returning again to the approximately upright state is performed 1 to 15 times preferably, 2 to 10 times more preferably, and 3 to 7 times even more preferably. Since the viscosity of the first agent 1 and the second agent 2 used for the present invention is much lower than that of a gel-like or cream-like agent type, the homogeneous liquid mixture 3 can be easily obtained even with the above-mentioned slow shaking of the pump foamer container 30. Here, being different from a technology disclosed in Patent Literature 3 described above, in the present invention, bubbling of the liquid mixture 3 does not cause any problem when obtaining the liquid mixture 3 by mixing the first liquid 1 and the second liquid 2. Since the air chamber of the pump foamer is structured to introduce air from the outside, bubbles do not enter to the air chamber even when content liquid is bubbled and bubbles are congested in the bottle. The above is because foam quality is not decreased accordingly.

The above embodiment is advantageous in that mixing operation can be effectively performed and that volume of the entire hair beauty preparation kit can be reduced.

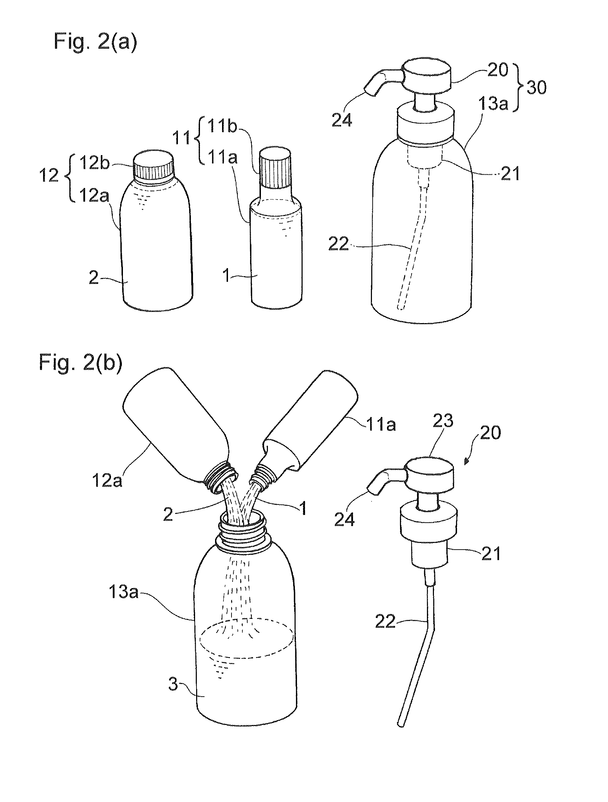

FIGS. 2(a) and 2(b) schematically illustrate a usage method of another embodiment of the hair beauty preparation kit of the first invention. Similarly to the hair beauty preparation kit illustrated in FIG. 1, the hair beauty preparation kit illustrated in the drawing includes the first container 11 and the second container 12. The first agent 1 and the second agent 2 are filled in the containers 11, 12, respectively. In addition to the above, the hair beauty preparation kit of the present embodiment includes a pump foamer container 30. In this manner, in the hair beauty preparation kit of the present embodiment, the pump foamer container 30 is formed separately from the first container 11 and the second container 12. The pump foamer container 30 includes a container body 13a and the pump foamer 20 which is screwed to a mouth portion of the container body 13a. In a state before usage of the hair beauty preparation kit, the container body 13a of the pump foamer container 30 is empty. It is preferable that the container body 13a of the pump foamer container 30 has a shape which is similar to the shape of the container body 12a of the second container 12 in the above-mentioned embodiment.

In a case of using the hair beauty preparation kit illustrated in FIGS. 2(a) and 2(b), first, the first agent 1 filled in the first container 11 and the second agent 2 filled in the second container 12 are poured into the container body 13a of the pump foamer container 30 and the first agent 1 and the second agent 2 are mixed. The mixing method may be similar to the method described in relation to the above-mentioned embodiment. Next, the pump foamer 20 is attached to the container body 13a of the pump foamer container 30 which stores the liquid mixture 3 to complete the pump foamer container 30. Similarly to the above-mentioned embodiment, the present embodiment is advantageous in that mixing operation can be effectively performed and that volume of the entire hair beauty preparation kit can be reduced owing to integration of the pump foamer container 30 with the container body 13a. In addition, according to the present embodiment, the first agent 1 filled in the first container 11 and the second agent 2 filled in the second container 12 can be used respectively in small quantity, so that one hair beauty preparation kit can be used in plural times. Therefore, there is an advantage of being economical, as well. In this case, the first container 11 and the second container 12 may be marked with a scale as a guide for usage quantity. Alternatively, the first agent 1 and the second agent 2 may be poured into the container body 13a of the pump foamer container 30 after being measured with a container for measuring (not illustrated). Further, since the container body 13a is empty in an initial state, one or both of the first container 11 and the second container 12 can be stored in the container body 13a when the container body 13a is larger compared to the first container 11 and the second container 12. Accordingly, volume of the entire kit can be reduced. Further, the larger the container body 13a is, the more heat is conducted to the container body. Accordingly, heat due to exoergic reaction during mixing of the first agent and the second agent can be expected to be effectively released.

As a first alternative method of the operation illustrated in FIG. 2(a), it is possible to pour the first agent 1 filled in the first container 11 and the second agent 2 filled in the second container 12 into a separately-prepared container (not illustrated) and to perform operation of mixing the first agent 1 and the second agent 2 in the container. In a case that the container is sharply shaken or the container is to be in an inverted state or a falling-over state for mixing the first agent 1 and the second agent 2, it is preferable that a mouth portion of the container is hermetically closed with a cover body (not illustrated). Liquid mixture 3 obtained through the mixing is transferred into the container body 13 of the pump foamer container 30. Similarly to the operation illustrated in FIG. 2(a), this operation has an advantage of being economical owing to that one hair beauty preparation kit can be used in plural times.

As a second alternative method of the operation illustrated in FIG. 2(a), it is also possible to perform operation of mixing the first agent 1 and the second agent 2 by inverting the container body 11a of the first container 11, inserting the mouth portion thereof to the container body 12a of the second container, and pouring the first agent 1 filled in the container body 11 a of the first container 11 into the container body 12a of the second container 12. In this case as well, liquid mixture 3 obtained through the mixing is only required to be transferred into the container body 13 of the pump foamer container 30. In a case that the container is sharply shaken or the container is to be in an inverted state or a falling-over state for mixing the first agent 1 and the second agent 2, it is preferable that the mouth portion of the container body 12a of the second container 12 is hermetically closed with the cover body 12b.

Compared to the first alternative method and the second alternative method described above, the operation illustrated in FIG. 2(a) is preferable in having an advantage that (i) volume of the entire kit can be reduced while reducing the number of structural elements of the hair beauty preparation kit and (ii) the number of processes from mixing operation to foam discharging can be reduced.

After the mixing operation is completed, foam of the liquid mixture 3 is discharged by depressing the pump head 23 of the pump foamer container 30 in any embodiment. For foam discharging, it is preferable that the pump head 23 is completely depressed in each depression operation of the pump head 23 from a viewpoint of capability to discharge large quantity of foam with the less operation number.

When discharging foam, the pump foamer container 30 is placed on a flat base and foam 4 of the liquid mixture 3 is discharged by depressing the pump head 23 with one hand under the above conditions, as illustrated in FIG. 3(a) for example. Here, it is possible to receive the discharged foam 4 with an instrument such as a brush, comb or the like or with another hand. It is preferable to receive the foam 4 with another hand from a viewpoint of capability of performing smooth applying operation. In a case of using a hand, it is preferable to put a glove thereon. The pump foamer container 30 illustrated in the drawing is a floor-standing type. However, as described above, there arises no problem in using a handy type as the pump foamer container 30.

Here, as an alternative method of the operation illustrated in FIG. 3(a), it is possible that a pump foamer container body has approximately rectangular shape (a cuboid having a bottom face with approximately rectangular shape, especially approximately square shape) as illustrated in FIG. 3(b). In a case of receiving foam with one hand while pressing the pump head with the other hand, force is applied to the pump foamer only by the other hand. Accordingly, there is a fear that stability becomes insufficient owing to that the container 30 is supported only by the flat base. Even in such an unstable state, when the bottom face of the pump foamer container body has an approximately rectangular or square shape, especially approximately square shape, the container 30 is less likely to fall even with repeated operation of pushing the pump head. In addition, a storage efficiency in a storage box is increased as the entire dye agent kit. Here, "approximately" denotes to allow some modification in shape such as chamfering corners to some extent.

From a viewpoint to reduce a possibility of falling during depression operation of the pump head and to improve a storage efficiency of the entire kit, it is preferable that the area of the bottom face of the pump foamer container body is in a range of 20 to 60 cm², 30 to 55 cm² more preferably, and 40 to 50 even more preferably.

Subsequently, the received foam 4 is applied to hair. From a viewpoint that dyeing can be performed evenly and that a sufficient dyeing effect is obtained while preventing liquid dripping, it is preferable that hairdressing is not applied to hair to which dye agent composition is applied just before dyeing procedure. Further, from a viewpoint that dyeing can be performed evenly and that a sufficient dyeing effect is obtained while preventing liquid dripping without attenuating the liquid mixture, dry hair is preferable. In a case of performing washing right before dyeing procedure, it is preferable that hair is dried until starting the dyeing procedure. Drying hair denotes liquid mainly being water attached by washing is removed at least to the extent of not being fallen in a natural state. Specifically, it is preferable of being in a towel dry state or a dry state with a dryer. For applying the foam 4 to hair, it is possible to use an instrument such as brush and comb. Alternatively, it is also possible to apply the hand-received foam 4 by directly using the hand. When the latter of the applying methods is adopted, there is an advantage in that the foam 4 can be easily applied to hair evenly from roots to tips thereof. Further, since the present invention does not require blocking operation, it is possible to smoothly apply the foam 4 of the liquid mixture 3 to unblocked hair with the latter of the methods.

In a case of adopting either of the above-mentioned methods, for applying the foam 4 to hair, it is preferable that operation to discharge the foam 4 with 1 to 5 times of depression of the pump head 23 after the foam 4 discharged with 1 to 5 times of depression of the pump head 23 is applied to hair is repeated. The number of times to depress the pump head 23 per repetition is 1 to 4 times more preferably, 1 to 3 times even more preferably, 1 to 2 times even more preferably, and 1 time most preferably. As described above, since the pump foamer container 30 used in the present invention is set to have discharge mass per single depression operation of the pump head 23 to be larger than that of a traditional pump foamer container, the foam 4 received by hand becomes sufficient in quantity when the number of such times of depression operation is performed. Further, since the discharge mass per single depression operation is set large, there is an advantage that applying quantity of foam per repetition can be enlarged and that anybody can perform dyeing beautifully with easy and even spreading.

The foam 4 may be applied to hair in a range of entire hair or only a specific section.

It is preferable that the foam 4 is rubbed using fingers like shampooing so that the foam 4 is spread to roots of hair sufficiently and evenly after specific quantity of the foam 4 (formed of the liquid mixture 3) is applied to hair by necessary quantity or at a midpoint of applying the entire necessary quantity of the foam 4. Since the foam 4 is foamed again with the rubbing even when being lessened, it is possible to prevent the liquid mixture 3 from falling from hair as well. In this case, it is preferable to perform rubbing slowly to prevent hair from tangling or to prevent the foam 4 from spattering. Rubbing operation may be performed in one time continuously or in plural times intermittently. After the foam 4 is rubbed as described above, it is left as it is for 3 to 60 minutes preferably, and 5 to 45 minutes more preferably. With the above, hair is dyed or bleached with the applied liquid mixture 3. Then, after lapse of a predetermined time, the liquid mixture 3 applied to hair is washed away with water and hair dyeing or bleaching operation is completed. After the liquid mixture 3 is washed away with water, it is possible to apply shampoo and/or conditioner as required.

According to the above-mentioned hair dyeing or bleaching method, sufficient quantity of foam 4 necessary for hair dyeing or bleaching can be discharged even with less number of times of discharge operation of the foam 4. Therefore, the operation of hair dyeing or bleaching can be smoothly performed. Further, hair dyeing or bleaching can be performed evenly from roots to tips of hair even without performing special operation such as blocking.

Next, description will be performed on the first agent 1 and the second agent 2 used in the present invention. The alkaline agent to be contained in the first agent 1 may adopt ammonia, alkanolamine such as monoethanolamine, sodium hydroxide, potassium hydroxide, or the like. Further, it is also possible to add, appropriately as a buffering agent, ammonium salt such as ammonium hydrogen carbonate and ammonium chloride, carbonate such as potassium carbonate and sodium hydrogen carbonate, or the like. A concentration of the alkaline agent is appropriately set so that pH of the liquid mixture 3 of the first agent 1 and the second agent 2 is in a range of 8 to 12 preferably, and 9 to 11 more preferably.

Meanwhile, a concentration of hydrogen peroxide contained in the second agent 2 is set to be in a range of 1% to 9% by mass preferably and 3% to 6% by mass more preferably. Then, a concentration of hydrogen peroxide in the liquid mixture 3 of the first agent 1 and the second agent 2 is set to be in a range of 1% to 6% by mass preferably and 2% to 5% by mass more preferably. For suppressing resolution of hydrogen peroxide, pH of the second agent 2 is set to be in a range of 2 to 6 preferably and 2.5 to 4 more preferably.

It is preferable that water functions as a main medium for both of the first agent 1 and the second agent 2.

Here, at least one of the first agent 1 and the second agent 2 contains a foaming agent. Accordingly, when the liquid mixture 3 of the first agent 1 and the second agent 2 is discharged from the pump foamer container 30, the liquid mixture 3 can be easily foamed and the foam can be maintained. Although anything may be adopted as the foaming agent as long as having a foaming property, a surface-active agent is preferable. Examples of the surface-active agent include a nonionic surface-active agent, an anionic surface-active agent, a cationic surface-active agent, an amphoteric surface-active agent, and a semipolar surface-active agent. Among the above, it is preferable to use an anionic surface-active agent or an amphoteric surface-active agent.

For example, the anionic surface-active agent may adopt a sulfate ester surface-active agent such as alkyl sulfate; a carboxylic acid surface-active agent such as fatty acid salt, polyoxyethylene alkyl ether carboxylate, N-acylamino acid salt and succinic acid alkyl salt; a phosphate ester surface-active agent such as alkyl phosphoric salt and alkyl ether phosphoric salt; and a sulphonic acid surface-active agent such as sulfosuccinate, isethionic acid salt, taurine salt, alpha olefin sulphonate and alkane sulphonate. Preferable examples thereof include alkyl sulfate, polyoxyethylene alkyl ether sulfate, polyoxyethylene alkyl ether carboxylate, and N-acylamino acid salt.

As the amphoteric surface-active agent, it is preferable to use fatty acid amide propyl betaine, alkylcarboxy methyl hydroxyethyl imidazolinium betaine, alkyldimethyl aminoacetic acid betaine, sulfo betaine, or the like.

The nonionic surface-active agent may adopt polyoxyethylene alkyl ether, alkyl polyglucoside, alkyl glyceryl ether, alkyl alkanolamide, or the like. Among the above, alkyl polyglucoside or polyoxyethylene alkyl ether is preferable. In a preferable example of alkyl polyglucoside, the carbon number of alkyl group is in a range of 8 to 14 and the condensation degree of glucoside is in a range of 1 to 2 on average. In a preferable example of polyoxyethylene alkyl ether, the carbon number of alkyl group is in a range of 10 to 18 and the condensation degree of polyoxyethylene is in a range of 5 to 40 on average.

The cationic surface-active agent may adopt alkyl chloride trimethylammonium, dialkyl chloride dimethylammonium, or the like.
The semipolar surface-active agent may adopt alkylaminoxide, or the like.

Two or more surface-active agents may be used in combination. To form foam which is easy to be applied to and be fit to hair, contained quantity of a surface-active agent in the liquid mixture 3 of the first agent 1 and the second agent 2 is in a range of 0.1% to 15% by mass preferably, 0.2% to 10% by mass more preferably, and 0.5% to 7% by mass even more preferably.

In a case that the two-part composition is for hair dyeing, examples of dye contained in the first agent 1 include oxidation dye and direct dye. Examples of the oxidation dye include a dye precursor such as para-phenylenediamine, para-aminophenol, toluene-2,5-diamine, N,N-bis (2-hydroxyethyl) para-phenylenediamine, 2-(2-hydroxyethyl) para-phenylenediamine, 4-amino-3-methylphenol, 6-amino-3-methylphenol, ortho-aminophenol and 1-hydroxyethyl-4,5-diaminopyrazole, and a coupler such as resorcin, 2-methylresorcin, meta-aminophenol, para-amino ortho-cresol, 5-(2-hydroxyethylamino)-2-methylphenol, meta-phenylenediamine, 2,4-diaminophenoxy ethanol and 1-naphthol. Examples of the direct dye include para-nitro ortho-phenylenediamine, para-nitro meta-phenylenediamine, basic yellow 87, basic orange 31, basic red 12, basic red 51, basic blue 99, acid orange 7, or the like.

To improve foam-lasting of foam of the liquid mixture 3 discharged from the pump foamer container 30 and to suppress occurrence of liquid dripping due to foam breakage after the foam is applied to hair, it is preferable that at least one of the first agent 1 and the second agent 2 contains higher alcohol. Preferably, the higher alcohol has the carbon atoms of 14 to 24. Examples thereof include myristyl alcohol, cetyl alcohol, stearyl alcohol, behenyl alcohol, isostearyl alcohol, and oleyl alcohol. Two or more thereof may be used in combination. Against the entire composition for hair dyeing after mixing of the first agent 1 and the second agent 2, alcohol is contained to be in a range of 0.01 % to 3% by mass preferably, 0.1 % to 2% by mass more preferably, 0.2% to 1% by mass even more preferably, and 0.3% to 0.8% by mass even more preferably.

In addition, the first agent 1 and the second agent 2 may contain a variety of additive agents as needed. For example, to prevent a scalp from being irritated by irritating constituent such as hydrogen peroxide condensed as moisture being evaporated after the liquid mixture 3 of the first agent 1 and the second agent 2 is applied to hair, it is preferable to add a non-volatile hydrophilic solvent such as polyols and alkyl ethers. Here, to form foam having firm foam quality and being easily spread from roots to tips of hair without being dripped, and further, to provide a conditioning effect to hair, it is also preferable to contain amphoteric or cationic polymer or the like. It is possible to appropriately contain essence, an ultraviolet absorptive agent, a sequestering agent such as edetic acid, a disinfecting agent, an antiseptic agent such as methyl parahydroxybenzoate, a stabilizing agent such as dibutylhydroxytoluene, 1-hydroxyethane-1,1-diphosphonic acid and oxyquinoline sulfate, an organic solvent such as ethanol, benzyl alcohol and benzyloxyethanol, a water-soluble polymer such as hydroxyethyl cellulose, a moisturizing agent, and the like.

A viscosity (at 25°C) of the first agent 1 is in a range of 1 to 50 mPa·s preferably, 3 to 40 mPa·s more preferably, and 5 to 30 mPa·s even more preferably. A viscosity (at 25°C) of the second agent 2 is in a range of 1 to 300 mPa·s preferably, 3 to 200 mPa·s more preferably, and 5 to 100 mPa·s even more preferably. A viscosity (at 25°C) of the liquid mixture 3 of the first agent 1 and the second agent 2 is set to be in a range of 1 to 300 mPa·s, 1 to 100 mPa·s preferably, 3 to 60 mPa·s more preferably, and 5 to 50 mPa·s even more preferably. Here, viscosity denotes a value obtained with a B-type rotating viscometer (manufactured by Tokyo Keiki INC., Model TV-10) using a rotor No. 1 after the rotor is rotated for one minute. The measurement is performed at a rotation speed being 60 rpm for 100 mPa·s or lower of a measurement target, being 30 rpm for a range of 100 to 200 mPa·s thereof, and being 12 rpm for a range of 200 to 500 mPa·s thereof. Viscosity of each of the first agent 1, the second agent 2, and the liquid mixture 3 is measured at 25°C in a constant-temperature reservoir. In a case of the liquid mixture 3, measurement is performed right after mixing and temperature variation due to reaction heat is not considered. Setting the viscosity of the liquid mixture 3 in the above-mentioned range is effective in that the foam-like liquid mixture 3 discharged from the pump foamer container 30 can be easily actualized to have foam volume (gas-liquid mixing ratio) being easily applicable to hair without having temperature dependency and that dripping of the liquid mixture can be suppressed during being left on hair after being applied to hair. Here, in view of easiness of the agent to be fit to and to be applied to hair, the gas-liquid mixing ratio is in a range of 7 to 40 mL/g preferably, and 10 to 30 mL/g more preferably. The gas-liquid mixing ratio of the above is measured as follows.

The first agent and the second agent which are adjusted to be room temperature are poured into the container body of the pump foamer container and mixed evenly. Foam is discharged to fill a container having volume of 50 mL after a lapse of one minute from pouring the first agent and the second agent into the same container. Then, foam of 50 mL is obtained by eliminating overflow with leveling. Mass of the discharged (stored) foam is obtained by measuring mass of the container storing the foam. The gas-liquid mixing ratio (mL/g) can be obtained by dividing the volume being 50 mL by the mass (g) of the discharged foam.

As a method to adjust viscosity of the liquid mixture 3 of the first agent 1 and the second agent 2 to be in the above-mentioned range, it is possible to simply adopt a method to add a hydrophilic solvent such as ethanol to the first agent 1 or the second agent 2 or to appropriately adjust a type or additive quantity of the above-mentioned surface-active agent, polyols, or higher alcohol.

The mixture ratio of the first agent 1 and the second agent 2 may be appropriately determined in accordance with color, brightness and the like obtained from the dye agent or the bleach agent. Specifically, the ratio of the first agent and the second agent by mass is in a range of 3:1 to 1:3 preferably, and 2:1 to 1:2 more preferably.

The total quantity of the first agent 1 and the second agent 2 depend on length of hair to be applied, whether being applied to hair entirely or partially, whether being used in separately or in full at a time. As one entire hair beauty preparation kit, the total quantity is in a range of 50 to 1000 mL preferably, 60 to 500 mL more preferably, 70 to 300 mL even more preferably, and 80 to 200 mL even more preferably.

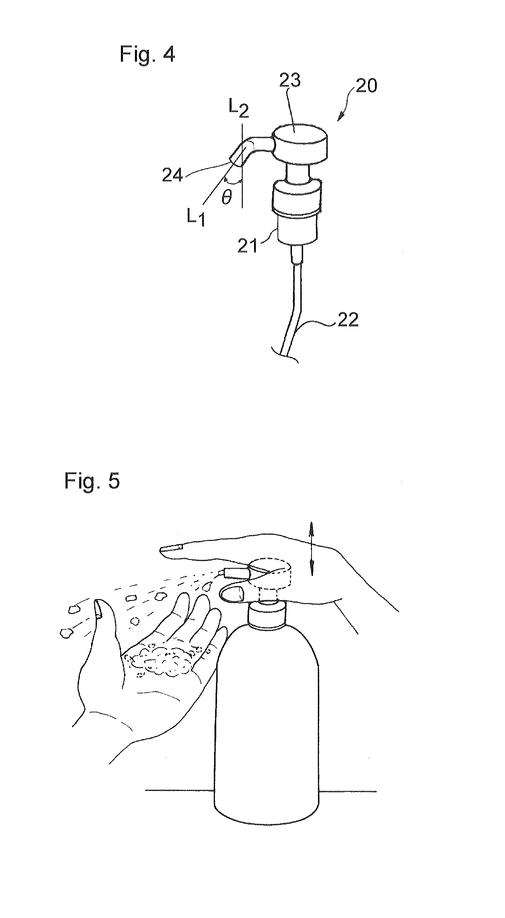

According to a hair beauty preparation kit of a second invention, the discharge port 24 of the pump foamer container 30 is oriented downward in an upright state of the pump foamer container 30 in the hair beauty preparation kit of the first invention, as illustrated in FIG. 4. Specifically, it is preferable that an angle θ between a perpendicular line L₁ of an opening face of the discharge port 24 and the vertical direction L₂ is in a range of 0° to 70° preferably, 10° to 60° more preferably, and 20° to 50° even more preferably. Setting the direction of the discharge port 24 as described above provides an advantage described below.

Generally, a discharge port is oriented in the horizontal direction or a direction close thereto in most cases, as illustrated in FIG. 5 for example. This is because a high production efficiency for shaping a discharge port section with a mold is obtained when the discharge port is oriented in the horizontal direction or a direction being close thereto. Further, traditionally, a product having small mass per one discharge has no problem even through a discharge port is oriented in the horizontal direction or a direction being close thereto.

In contrast, in the pump foamer container 30 in the hair beauty preparation kit of the present invention, discharge mass of the foam 4 per single depression operation is set large. In a case that the discharge port is oriented in the horizontal direction or a direction being close thereto as illustrated in FIG. 5 for example (although a state without gloves is illustrated only in this drawing for convenience sake, it is intended to be with gloves), when the pump head 23 is unintentionally depressed sharply, there may arise a problem that the foam 4 spatters as not being received successfully by a palm as the foam 4 being sharply discharged as illustrated in the drawing. Such a problem is specific to the present invention in which discharge mass of the foam 4 per single depression operation is set large as a kit of a dye agent or a bleach agent while the problem does not occur in traditional art, for example, in technologies described in Patent Literatures 1 to 3. To solve such a problem, in the second invention, the discharge port 24 is oriented as described above as being contrary to traditional common sense. With the above, even when the foam 4 is sharply discharged, the foam 4 can be received successfully by a palm as illustrated in FIG. 3. Normally, the pump head 23 and the discharge port 24 are formed as an integrated component. Here, for example, setting the angle θ between the perpendicular line L₁ of the opening face of the discharge port 24 and the vertical direction L₂ into the above-mentioned range can be performed by attaching a component separate from the pump head 23.

According to a hair beauty preparation kit of a third invention, the area of the discharge port 24 of the pump former 20 of the pump foamer container 30 is set larger than the area of a discharge port of a traditional pump foamer in the hair beauty preparation kit of the first invention. Specifically, it is preferable that the area of the discharge port 24 is set in a range of 45 to 200 mm², 55 to 150 mm² more preferably, and 65 to 100 mm² even more preferably. Setting the area of the discharge port 24 to be large as described above provides an advantage described below. As described above, in the pump foamer container 30 of the hair beauty preparation kit of the present invention, the discharge mass of the foam 4 per single depression operation is set large. When the pump head 23 is unintentionally depressed sharply, there may arise a problem that the foam 4 spatters and is not received successfully by a palm as the foam 4 being sharply discharged. Such a problem is specific to the present invention in which discharge mass of the foam 4 per single depression operation is set large as a kit of a dye agent or a bleach agent while the problem does not occur in traditional art, for example, in technologies described in Patent Literatures 1 to 3. To solve such a problem, in the third invention, the area of the discharge port 24 is set in the above-mentioned range in relation to the discharge mass of the foam 4 per single depression operation. With the above, since the foam 4 is not discharged so sharply even when the pump head 23 is sharply depressed, the foam 4 can be received successfully by a palm.

According to a hair beauty preparation kit of a fourth invention, a maximum load while the pump head 23 of the pump foamer 20 of the pump foamer container 30 is completely depressed is set smaller than a depressing load of a pump head of a traditional pump foamer in the hair beauty preparation kit of the first invention. Specifically, it is preferable that the maximum load for complete depression of the pump head 23 in a state that the liquid mixture of the first agent and the second agent is stored in the container body is set in a range of 10 to 25 N, 12 to 23 N more preferably, and 15 to 22 N even more preferably. A depressing load of a pump head of a traditional pump foamer is set large owing to common sense that the pump head is required to be returned to an original point rapidly and reliably without being stuck for continuous pushing of the pump head. Lessening thereof is contrary to common sense. Nevertheless, setting the depressing load of the pump head 23 to be small as described above in the present invention provides an advantage described below. As described above, in the pump foamer container 30 of the hair beauty preparation kit of the present invention, the discharge mass of the foam 4 per single depression operation is set large. Accordingly, sufficient quantity of the foam 4 can be received by a hand even with single depression operation. However, when a load during the depression operation is large, there may be a case to cause a problem that excessive force is required for the depression resulting in a large physical burden for a user. Further, when the load during the depression operation is large, there is a tendency that a user sharply performs depressing unintentionally. As described above, unintentionally sharp depression may cause a problem that the foam 4 spatters without being received successfully by a palm. Further, sharp depression may cause a problem of unstable depression operation. Accordingly, the above is not advantageous for usage of a dye agent. Such a problem is specific to the present invention in which discharge mass of the foam 4 per single depression operation is set large as a kit of a dye agent or a bleach agent while the problem does not occur in traditional art, for example, in technologies described in Patent Literatures 1 to 3. To solve such a problem, in the fourth invention, the load during depression operation is set to be in the above-mentioned range. With the above, since the depression operation can be performed with relatively small force, a physical burden of a user is lessened and discharged foam can be reliably received by one hand with the depression operation being stable and gentle regardless of intention. Here, setting the load during depression operation into the above-mentioned range can be performed by selecting material which has appropriate elasticity for a spring arranged in the pump head 23, and the like.

Measurement of the maximum load during depression of the pump head 23 of the pump foamer container 30 is performed as follows. First, liquid mixture of the first agent and the second agent adjusted to be at room temperature is poured into the container body of the pump foamer and is mixed evenly. After one minute passes from pouring of the first agent and the second agent into the single container, blind depression is performed several times. When foam becomes to be discharged stably, the pump head is depressed completely at a depression speed of 30 mm/sec using a pressing force tester "dynamic force processor F381" manufactured by Unipulse Corporation. The maximum load obtained during the depressing process is to be a measurement value.

According to a hair beauty preparation kit of a fifth invention, control is performed in the hair beauty preparation kit of the first invention on the time from when the depressed state of the pump head 23 of the pump foamer 20 of the pump foamer container 30 is released after the pump head 23 is completely depressed until the pump head 23 is returned to an original position before depressing of the pump head 23. Specifically, it is preferable that the time until the pump head 23 is returned to the original position before depressing of the pump head 23 in a state that the liquid mixture of the first agent and the second agent is stored in the container body is set to be relatively long time as being in a range of 0.2 to 3 seconds preferably, 0.4 to 2 seconds more preferably, and 0.6 to 1.5 seconds even more preferably. With a usual pump foamer container, a pump head is designed to be instantly returned to an original position before depression. The time setting in the present invention particularly has a technical meaning in relation to the fourth invention described above. That is, lessening the load during depression operation of the pump head 23 is lengthening the time until the pump head 23 is returned to an original position before depression, in other words. In general, lengthening the time until the pump head 23 is returned to the original position before depression is not advantageous as possibly causing delay of entire operation in relation to performing repeated operation to depress the pump head. In particular, it is all the more because difference of the time in the above-mentioned range from traditional time is clearly recognizable for a general user. However, as described above, in the present invention, since discharge quantity of the foam 4 per single depression operation is set large as a kit of a dye agent or a bleach agent, sufficient quantity of the foam 4 is discharged with depression of the pump head 23. Accordingly, the foam 4 can be applied to hair sufficiently only with one time of depression operation. Therefore, time intervals from performing depression operation until performing the next depression operation are much longer than those of a traditional pump former container. Accordingly, it is not required to shorten the time from the pump head 23 is depressed until being returned to an original position before depression. In other words, even when it takes time until the pump head 23 is returned to the original position before depression, dyeing operation can be smoothly performed without causing delay on operation of dyeing or bleaching. Further, in relation to the fourth invention described above, since the load during depression operation of the pump head 23 is lessened, dyeing operation can be performed smoothly all the more.

Measurement is performed as follows for the time from when depressed state of the pump head 23 is released after the pump head 23 is completely depressed until the pump head 23 is returned to the original position before depression in a state that the liquid mixture of the first agent and the second agent is stored in the container body. First, the pump foamer is prepared in a state that sufficient quantity of the liquid mixture exists in the container body. Video recording is started on the pump foamer container from an arbitral time point before releasing the depressed state of the pump head 23 after the pump head 23 is completely depressed. The video recording is stopped after the depressed state is released and the pump head 23 is stopped as being returned to the original position before depression. After recording, frame-by-frame playback is performed with the recorded image. The targeted time is obtained through the number of frames in a state that the pump head is moving.

According to a hair beauty preparation kit of a sixth invention, the air chamber 21 of the pump foamer 20 of the pump foamer container 30 exists outside the container body 30a in the hair beauty preparation kit of the first invention, as illustrated in FIG. 6. Being different from the embodiment illustrated in FIG. 6, the air chamber of the pump foamer is located inside the container body in the embodiments described above. Locating the air chamber outside the container body as in the present embodiment provides an advantage described below. As described above, in the pump foamer container 30 of the hair beauty preparation kit of the present invention, the discharge mass of the foam 4 per single depression operation is set large. Further, as described above, volume of the air chamber 21 of the pump foamer is required to be large for increasing discharge mass of the foam 4. Here, when volume of the air chamber 21 is large in a case that the air chamber 21 is stored in the container body of the pump foamer container 30, volume of the container body is necessarily required to be large. This may cause a problem that volume of the hair beauty preparation kit is increased. Such a problem is specific to the present invention in which discharge mass of the foam 4 per single depression operation is set large as a kit of a dye agent or a bleach agent while the problem does not occur in traditional art, for example, in technologies described in Patent Literatures 1 to 3. To solve such a problem, in the sixth invention, the air chamber 21 of the pump foamer 20 is located outside the container body 30a. With the above, the entire volume of the pump foamer container 30 can be set small and further the entire volume of the kit for dyeing or bleaching can be set small even when discharge mass of the foam 4 per single depression operation is increased. Further, the inside of the container body is simplified owing to that the air chamber does not exist in the container body of the pump foamer container 30. Accordingly, mixing of the first agent 1 and the second agent 2 can be performed more easily.

A specific structure of the pump foamer container 30 in the present embodiment is as follows. That is, the pump foamer container 30 includes a container body 30a which stores content liquid and a pump foamer 20 which is attached to a mouth-neck portion 312 of the container body 30a. The pump foamer 20 includes a pump head 314 which is reciprocatable and which is connected to a piston portion 315. When the pump head 314 is depressed, the pump foamer 20 opens a first flow passage 320 which feeds content liquid to a mixing chamber 319 from a pressurized small-diameter liquid chamber 317 of a cylinder portion 316 and a second flow passage 321 which feeds air to the mixing chamber 319 from a pressurized large-diameter air chamber 21 of the cylinder portion 316. The liquid mixture and air fed to the mixing chamber 319 are discharged as being mixed and foamed at a foaming flow passage 323 from the mixing chamber 319 to the discharge port 24.

The piston portion 315 includes a cylindrical piston member 324 which has a lower end section slid along an inner circumferential face of the liquid chamber 317 as penetrating a center section of the air chamber and an air piston member 325 which is slid along an inner circumferential face of the air chamber 21 as being arranged to be protruded outward in the radial direction from the cylindrical piston member 324. Here, the pump foamer 20 is integrally attached to the mouth-neck portion 312 in a state that the air chamber 21 is arranged outside while a bottom circumferential portion 321 a of the air chamber 21 is arranged above the mouth-neck portion 312 of the container body 30a and an outer circumferential face 321b of the air chamber 21 is arranged along an outer circumferential face 312b of the mouth-neck portion 312.

A male thread portion is formed at the outer circumferential face 321b of a bottom part of the air chamber 21. The pump foamer 20 is integrally attached to the mouth-neck portion 312 via a joint sleeve member 326 having a female thread portion formed at an inner circumferential face to be screwed with both of the male thread portion of the air chamber 21 and a male thread portion formed at the outer circumferential face 312b of the mouth-neck portion 312.

In the above, the present invention is described based on preferable embodiments. However, the present invention is not limited to the embodiments and forms which are appropriately modified from the above-mentioned embodiments within the scope of an ordinary-skilled person in the art fall in the scope of the present invention.

### [Test examples]

The following tests were performed with a first agent and a second agent of "Prettia Funwari Foam color" (manufactured by Kao Corporation) (color: custard brown, viscosity of liquid mixture of the first agent and the second agent at 25°C: 12 mPa·s, total mass of the liquid mixture: 100 g, foamer container: squeeze foamer).

### [Test Example 1]

The first agent and the second agent were poured into a container body of a pump foamer G3 of a floor-standing type manufactured by Daiwa Kan Company (design specifications: discharge mass being 3.0 g, discharge volume being 30 cm³, a gas-liquid mixing ratio being 10 mL/g, a stroke of a pump head being 20 mm, an angle between a perpendicular line of an opening face of a discharge port and the vertical direction being 5°, the sectional area of the discharge port being 50 mm², a standard-equipped bottle body being cylindrical with the bottom area of 44.2 cm²). The pump head was fully depressed at a depression speed of 30 mm/sec using a pressing force tester "dynamic force processor F381" manufactured by Unipulse Corporation. The maximum load was 29.0 N (16.9 N when the container was empty). The time required until the pump head was returned to an original position after releasing the depressing force was 0.13 sec. Regarding foam actually discharged with single depression of the pump head, mass was 3.1 g, foam volume was 31 cm³, and a gas-liquid mixing ratio was 10 mL/g. The foam is just right for being held by one hand with one or two times of depression operation of the pump head without dropping from the hand.

### [Test Example 2]

A test was performed similarly to Test Example 1 other than pushing the pump head with a dominant hand. Through several times of pushing of the pump head, there was an unintentional tendency to have a higher speed (a depression speed about 50 mm/sec in approximation) compared to the depression speed in Test Example 1 owing to the higher depression pressure as well. With quick depressing, discharged foam dropped from a receiving palm frequently.

### [Test Example 3]

A separate component was attached to the discharge port of the pump foamer G3 manufactured by Daiwa Kan Company and an angle between a perpendicular line of an opening face of the discharge port and the vertical direction was adjusted to 30°. A test was performed similarly to Test Example 2 other than the above. There was no dropping of foam from a hand even with quick depression of the pump head (a depression speed about 50 mm/sec in approximation).

### [Test Example 4]

A spring in the pump foamer G3 manufactured by Daiwa Kan Company was removed and a spring obtained from Misumi Corporation (model number: WY10-65, diameter: 10 mm, length: 65 mm) was attached instead. The test was performed similarly to Test Example 1 other than the above. The maximum load was 19.7 N (9.9 N when the container was empty). The time required until the pump head was returned to an original position after releasing the depressing force was 0.77 sec. Regarding foam actually discharged with single depression of the pump head, mass was 2.8 g, foam volume was 31 cm³, and a gas-liquid mixing ratio was 11 mL/g. Compared to Test Example 1, the returning speed of the pump head was slow as being recognizable at a glance and seemed unsuitable for repeated usage based on common sense. However, when being used for hair dyeing, it appeared user-friendly actually.

### [Test Example 5]

A floor-standing type pump foamer E3 manufactured by Daiwa Kan Company was used as the container body. There is a plurality of design specifications among E3. The present test example was performed with design specifications as discharge mass being 1.6 g, discharge volume being 20 cm³, a gas-liquid mixing ratio being 12.5 mL/g, a stroke of a pump head being 20 mm, an angle between a perpendicular line of an opening face of a discharge port and the vertical direction being 5°, the sectional area of the discharge port being 50 mm², a standard-equipped bottle body being cylindrical with the bottom area of 28.3 cm². The maximum load was 24.1 N (15.2 N when the container was empty). The time required until the pump head was returned to an original position after releasing the depressing force was 0.13 sec. Regarding foam actually discharged with single depression of the pump head, mass was 1.64 g, foam volume was 26 cm³, and a gas-liquid mixing ratio was 16 mL/g. The foam is just right for being held by one hand with about two times of depression operation of the pump head without dropping from the hand.

### [Test Example 6]

A test was performed similarly to Test Example 1 other than using a handy type pump foamer F2 manufactured by Daiwa Kan Company disclosed in an embodiment of Patent Literature 1 (Japanese Patent Application Laid-Open No. 2004-339216). The foamer has design specifications as discharge mass being 0.75 g, discharge volume being 10 cm³, a gas-liquid mixing ratio being 13.3 mL/g, a stroke of a pump head being 15 mm, an angle between a perpendicular line of an opening face of a discharge port and the vertical direction being 0°, the sectional area of the discharge port being 38 mm², and a standard-equipped bottle body being cylindrical with the bottom area of 17.3 cm². The maximum load was 24.4 N (23.1 N when the container was empty). The time required until the pump head was returned to an original position after releasing the depressing force was 0.07 sec. Regarding foam actually discharged with single depression of the pump head, mass was 0.74 g, foam volume was 10 cm³, and a gas-liquid mixing ratio was 14 mL/g. The foam is just for being held by one hand in quantity finally with about five times of depression operation of the pump head. Although the maximum load is slightly smaller than other test examples, a plural times of continuous depression is tiring as a result of so many times of depression and further being a handy type.

### [Test Example 7]

A test was performed similarly to Test Example 1 other than using a floor-standing type pump foamer container bundled in a two-part foam-like hair dye agent with a pump foamer "Dream 17 Musiran" (commissioning manufacturer: Dong-an Biotechnology Industrial Corporation) commercially available in Republic of China. The above had a stroke of a pump head being 17 mm, an angle between a perpendicular line of an opening face of a discharge port and the vertical direction being 0°, the sectional area of the discharge port being 36 mm², a bottle body being like an elliptic cylinder with the bottom area of 15.8 cm². The maximum load was 33.8 N (32.5 N when the container was empty). The time required until the pump head was returned to an original position after releasing the depressing force was 0.07 sec. Regarding foam actually discharged with single depression of the pump head, mass was 0.78 g, foam volume was 9.4 cm³, and a gas-liquid mixing ratio was 12 mL/g.

### [Test Example 8]

A test was performed similarly to Test Example 1 other than using a handy type pump foamer container bundled in a two-part foam-like hair dye agent with a pump foamer "Pretty Color" (manufactured by Legal Practice Science and Technology Corporation) commercially available in Republic of China. The above had a stroke of a pump head being 15 mm, an angle between a perpendicular line of an opening face of a discharge port and the vertical direction being 0°, the sectional area of the discharge port being 20 mm², a bottle body being cylindrical with the bottom area of 17.9 cm². The maximum load was 24.9 N (23.3 N when the container was empty). The time required until the pump head was returned to an original position after releasing the depressing force was 0.10 sec. Regarding foam actually discharged with single depression of the pump head, mass was 0.76 g, foam volume was 8.4 cm³, and a gas-liquid mixing ratio was 11 mL/g.

### [Test Example 9]

A test was performed similarly to Test Example 1 other than using a handy type pump foamer container bundled in a two-part foam-like hair dye agent with a pump foamer "Bubble Hair Color" (manufactured by Guangzhou Wenya Daily-use Cosmetics Corporation) commercially available in People's Republic of China. The above had a stroke of a pump head being 15 mm, an angle between a perpendicular line of an opening face of a discharge port and the vertical direction being 0°, the sectional area of the discharge port being 28 mm², a bottle body being cylindrical with the bottom area of 15.2 cm². The maximum load was 30.1 N (29.8 N when the container was empty). The time required until the pump head was returned to an original position after releasing the depressing force was 0.07 sec. Regarding foam actually discharged with single depression of the pump head, mass was 0.69 g, foam volume was 9.0 cm³, and a gas-liquid mixing ratio was 13 mL/g.

## Claims

1. A method for hair dyeing or bleaching using a two-part hair dye or bleach kit comprising:
a two-part hair dye or bleach composition including a first agent which contains an alkaline agent and a second agent which contains hydrogen peroxide,
a container body in which liquid mixture of the first agent and the second agent is to be stored, and
a pump foamer which is to be attached to the container body and which discharges the liquid mixture in a foam,
wherein at least one of the first agent and the second agent contains a foaming agent,
a viscosity of the liquid mixture is in a range of from 1 to 300 mPa·s at 25°C,
a discharge mass of the pump foamer per single depression operation is 1 g or more, and
the method includes steps of:
A) mixing the first agent and the second agent in the container body;
B) discharging foam of the liquid mixture by depressing a pump head of a pump foamer container in which the pump foamer is attached to the container body;
C) applying the discharged foam of the liquid mixture to hair;
D) allowing the liquid mixture applied to hair to stand for 3 to 60 minutes; and
E) washing away the liquid mixture applied to hair.

2. The method for hair dyeing or bleaching according to claim 1,
wherein the kit includes a container body in which the first agent is filled, a container body in which the second agent is filled, the container body in which the liquid mixture of the first agent and the second agent is to be stored, and the pump foamer which is to be attached to the container body storing the liquid mixture, and
the pump foamer container is completed by attaching the pump foamer to the container body storing the liquid mixture.

3. The method for hair dyeing or bleaching according to claim 1,
wherein the kit includes a container body in which the first agent is filled, a container body in which the second agent is filled, and the pump foamer which is to be attached to the container body in which the second agent is filled, and
the pump foamer container is completed by pouring the first agent into the container body in which the second agent is filled, followed by attaching the pump foamer to the container body.

4. The method for hair dyeing or bleaching according to any one of claims 1 to 3,
wherein a discharge port of the pump foamer container is formed to have an angle θ between a perpendicular line of an opening face of the discharge port and the vertical direction being in a range of from 0° to 70° in a state that the pump foamer container is upright.

5. The method for hair dyeing or bleaching according to any one of claims 1 to 4,
wherein an area of a discharge port of the pump foamer container is in a range of from 45 to 200 mm².

6. The method for hair dyeing or bleaching according to any one of claims 1 to 5,
wherein a maximum load until a pump head of the pump foamer container is completely depressed in a state that the liquid mixture of the first agent and the second agent is stored in the container body is in a range of from 10 to 25 N.

7. The method for hair dyeing or bleaching according to any one of claims 1 to 6,
wherein a pump head of the pump foamer container takes time for from 0.2 to 3 seconds until being returned to an original position before depression when a depressed state of the pump head is released in a state that the liquid mixture of the first agent and the second agent is stored in the container body after the pump head is completely depressed.

8. The method for hair dyeing or bleaching according to any one of claims 1 to 7,
wherein the pump foamer discharges a mass of 8 g or less per single depression operation.

9. A two-part hair dye or bleach kit comprising:
a two-part hair dye or bleach composition including a first agent which contains an alkaline agent and a second agent which contains hydrogen peroxide;
a container body in which liquid mixture of the first agent and the second agent is to be stored; and
a pump foamer which is to be attached to the container body and which discharges the liquid mixture in a foam,
wherein a pump foamer container is completed by attaching the pump foamer to the container body,
at least one of the first agent and the second agent contains a foaming agent,
a viscosity of the liquid mixture is in a range of from 1 to 300 mPa·s at 25°C, and
a discharge mass of the pump foamer container per single depression operation is 1 g or more.

10. The two-part hair dye or bleach kit according to claim 9, further comprising:
a container body in which the first agent is filled;
a container body in which the second agent is filled;
the container body in which the liquid mixture of the first agent and the second agent is to be stored; and
the pump foamer which is to be attached to the container body,
wherein the pump foamer container is completed by attaching the pump foamer to the container body storing the liquid mixture of the first agent and the second agent.

11. The two-part hair dye or bleach kit according to claim 9, further comprising:
a container body in which the first agent is filled;
a container body in which the second agent is filled; and
the pump foamer which is to be attached to the container body in which the second agent is filled,
wherein the pump foamer container is completed by pouring the first agent into the container body in which the second agent is filled, followed by attaching the pump foamer to the container body.
